# EUROPEAN PATENT SPECIFICATION

(11) **EP 2 331 191 B1**
(45) Date of publication and mention of the grant of the patent: **20.03.2019**
(21) Application number: 09812163.5
(22) Date of filing: 02.09.2009
(51) Int. Cl.: A61M 39/02

(54) **CLOSED MALE LUER DEVICE FOR MINIMIZING LEAKAGE DURING CONNECTION AND DISCONNECTION**
GESCHLOSSENE LUER-VORRICHTUNG ZUR MINIMIERIUNG VON LECKAGE BEI ANSCHLUSS UND TRENNUNG
DISPOSITIF LUER MÂLE CLOS DESTINÉ À MINIMISER LES FUITES LORS D'UNE CONNEXION ET D'UNE DÉCONNEXION

(30) Priority: 05.09.2008 US 204962
(43) Date of publication of application: 15.06.2011
(73) Proprietor: CareFusion 303, Inc., San Diego, CA 92130 (US)
(72) Inventor: MANSOUR, George, M., Pomona CA 91766 (US); TRUITT, Tim, L., Orange CA 92869 (US); FRIED, Matthew, Paul, Ontario CA 91761 (US)
(74) Representative: Epping - Hermann - Fischer
(86) International application number: PCT/US2009/055728
(87) International publication number: WO 2010/028040

(56) References cited:
- WO-A1-00/20070
- WO-A1-2006/062912
- JP-A- 2006 102 254
- US-A1- 2003 060 804
- US-A1- 2004 217 315
- US-A1- 2004 227 120
- US-A1- 2006 118 749
- US-A1- 2007 021 721
- US-A1- 2007 073 270
- US-A1- 2007 088 325
- US-A1- 2007 106 244
- US-B2- 7 195 616
- US-B2- 7 329 249

## Description

### TECHNICAL FIELD

The present invention relates to medical connectors used in fluid delivery applications, and more specifically to connectors that minimize fluid leakage during connection and disconnection.

### BACKGROUND OF THE INVENTION

Medical connections are widely used in fluid delivery systems such as those used in connection with intravenous fluid lines, blood access, hemodialysis, peritoneal dialysis, enteral feeding, drug vial access, etc. Many prior art aseptic medical connections has been to puncture an elastomeric diaphragm or septum, which has one side in contact with the fluid, with a sharpened hollow hypodermic needle. The use of such hypodermic needles has been gradually decreasing as a result of both safety and cost considerations associated with infectious disease acquired from needle sticks. These connectors have been replaced with luer activated connectors which don't require hypodermic needles, but instead use an activator such as a luer on the end of a syringe or IV line to create a fluid path though a valve in a connector. The removal of the connector causes the valve to close when the line is disconnected. Such a system is described in United States Patent 5,569,235 to Ross et al..

Fangrow (US 2007/0088325 (Fangrow, Thomas F. Jr. (US) 19 April 2007)) discloses a luer connector comprising a housing with a hollow bore having first and second ends. The hollow bore also has a male luer tip and a tapering interior surface. The luer connector also comprises a rigid valve member configured to at least partially extend through the housing. The valve member has a first opened end and a second closed end. The valve member also comprises a passageway within the valve member and an outwardly extending flange near the second end adapted to seal the hollow bore at the second end of the housing when placed in contact with a tapering interior surface of the housing. The valve member further comprises at least one opening near the closed end of the valve member extending outward from the passageway through the valve member and at least one strut attached to the valve member. At least a portion of the strut extends substantially parallel to the central axis of the valve member. The luer connector also comprises a retaining member configured to couple the valve member and the housing and a sealing element disposed within the housing. The sealing element is configured to inhibit fluid communication through the hollow bore of the housing between the interior of the male luer tip of the housing and the first end of the housing.

Raybuck (US 2004/0227120 (Raybuck, John (US) 18 November 2004)) discloses a self-sealing male connector having a body, a resilient boot mounted over the body, and a male thread hub. The body is formed with an elongate post that extends to the distal end. The body includes a fluid flow lumen extending from its proximal end to its distal end. At its proximal end, the body may be formed as a female Luer connector, or may be directly connected to tubing, or other arrangements. The internal fluid flow lumen extends through the post to its distal end. The body includes a first groove in which is mounted the boot. In a second groove is mounted the thread cuff. The thread cuff is used to engage a female connector and through thread action, draw in into engagement with the post. As the female connector is further engaged with eh sealed male connector, the post protrudes through the boot and into fluid communication with the female fluid flow passage thus establishing a flow path through both connectors. The boot forms a seal with the female connector when engaged and returns to seal the male connector post and fluid flow passage within disconnected from the female connector.

Whitley (WO 2006/062912 (Whitley, Kenneth (US) 15 June 2006)) discloses a self-sealing male Luer connector (10) attaches to any standard female Luer valve to open a flow channel between the two. The self-sealing male Luer connector (10) includes a rigid housing (12) having a distal end (16) with a rigid male Luer connector and a proximal end (14) at which a proximal seal (82) is formed. The distal end (16) of the housing (12) includes a valve seat (70).
Located within the housing (12) is a resilient biasing member (34) that biases an actuator (36) into contact with the valve seat (70) to prevent fluid flow through the male connector (10). Upon engagement with a female connector, the actuator (36) is moved in the proximal direction to open the distal valve and then the proximal seal (82). A partial vacuum is formed within the male connector (10) upon disengagement with the female connector that draws any fluids on the external surface of the distal end (16) of the male Luer connector into the male tip.

Christensen (US 2007/0073270 (Cristensen, Marke A. (US) 29 March 2007)) discloses a catheter connection system. In one embodiment, a catheter connection system may include at least two components and a deformable sealing element positioned between the at least two components configured to allow, upon deformation, fluid flow through the at least two components. In another embodiment, the catheter connection system may include a sealing element positioned between at least two components, wherein the components are coupled to one another by a locking member.

Lopez (US 2007/0021721 (Lopez, George A. (US) 25 January 2007)) discloses a medical valve has a body and a flexible element. The body includes a wall structure defining an internal cavity having an inside and an outside. The body also has a proximal end and a distal end. The proximal end has an opening sufficiently large to receive a tip of a delivery end of a medical implement which transfers fluid through the delivery end. The body has a fluid escape space in its wall structure. The flexible element is adapted to be moved into a compressed state upon insertion of the tip of the medical implement into the opening. The flexible element is sufficiently resilient to return to a decompressed state upon removal of the tip of the medical implement from the opening. The fluid escape space is in fluid communication with the outside of the cavity when the seal is in its compressed state.

Ryan (US 2006/0118749 (Ryan, Dana Wm. (US) 8 June 2006)) discloses an improved needle-free intravenous injection port assembly is disclosed. Embodiments include a boot valve with a helical surface, a boot valve and septum which mate with mechanical interference, a spike with a rough outer surface coated with a lubricant, a septum having a shoulder and a single continuous swabbable surface, a septum and a boot valve which are pre-punctured, a septum with a frustroconical extension and a combination single piece septum and boot valve. The injection port assembly provides zero fluid displacement during coupling and uncoupling.

Ryan (WO 00/20070 (Ryan, Dana Wm. (US) 13 April 2000)) discloses a needleless IV injection port assembly (100) includes a spiked body (102) provided with a hollow spike (104), a female luer component, a flexible and resilient spike boot (108) having a tip (170) and spring portion (172), a centering member (110), and a resilient swabbable septum (112). When a separate male luer is positioned over a protuberence on the septum (112) and pushed into the female luer component, the male luer urges the septum (112) toward the spike (104), causing the spring portion (172) of the boot (108) to be compressed and the tip portion (170) of the boot to be pierced by the tip of the spike (104). The injection port causes minimal reflux, and the spike boot (108) and septum (112) provide a swabbable antibacterial double barrier over the spike (104).

Susumu (JP 2006-102254 (Susumu, Miyasaku (JP) 20 April 2006)) discloses a problem to be solved of: to obtain a connecting structure of an infusion line by which the proximity of an open hole can be constantly kept clean. A solution being: the connecting structure of the infusion line for passing a medical fluid is provided with an injection tube 2 consisting of an injection part 4 which is in the shape of a cylinder with bottom and has openings 5 in its peripheral wall at its end and an injection part 3 which is communicated with the inside of the injection part 4, an external cylinder part 7 in the shape of a cylinder with bottom which has a bottom 7a integrated with the injection tube 2 and is open toward its end for being fitted to a partner member 20 and a protective shutter 6 which is outwardly fitted to the injection part 4 in such a way that it blocks off the openings 5. When the protective shutter 6 is subjected to a pressure higher than a predetermined degree toward its base, it moves along the axis of the injection part 4 and releases the openings 5. When the pressure is relieved, it returns to its distal end and blocks off the openings 5.

Typical connectors and valves of this type, such as described by Ross, have many attributes that are not ideal in medical applications for delivery of fluids that could be harmful if contacted by the health care provider or the patient other than through the patient's intravenous ("IV") connection. Oncology drugs such as chemotherapy are examples of fluid that while beneficial to the patient as part of a treatment regimen could be extremely harmful to the health care provider if the chemotherapy drug were to come into contact with the skin of the health care provider or patient.

Traditional medical connectors require the health care provider to exercise great care on connection or disconnection due to the likelihood of the drug remaining inside the connector or dripping, particularly on disconnection when the connectors are primed with fluid. Some female connectors are designed to push fluid in the throat of the connector to the surface during disconnection. While this is desirable for asceptic connectors to provide a swabbable surface, it can result in fluid drips from the device on disconnection. Other connectors use a membrane with a septum that can also allow fluids to escape the connector.

What is needed is a connector for medical fluids that has standardized connections for use with existing medical connectors and also minimizes or eliminates drips on connection or disconnection.

### BRIEF SUMMARY OF THE INVENTION

The present invention concerns a medical connector according to claim 1 and a method of connecting a connector and an access device according to claim 9. Preferred embodiments are described in dependent claims 2 to 8 and 10 to 13.

### BRIEF DESCRIPTION OF THE DRAWINGS

For a more complete understanding of the present invention, reference is now made to the following descriptions taken in conjunction with the accompanying drawings, in which:
FIG. 1 is a perspective view of an embodiment of a male luer medical connector for fluid delivery according to the concepts described herein;
FIG. 2 is a sectional view of the male luer medical connector shown in Figure 1;
FIGs. 3A-3C are perspective views of embodiments of the components of the male luer medical connector shown in Figure 1; and
FIGs. 4A and 4B are sectional views of an embodiment of a male luer medical connector in accordance with the concepts shown herein and a deformable valve plug type medical connector in a disconnected state and a connected state respectively.

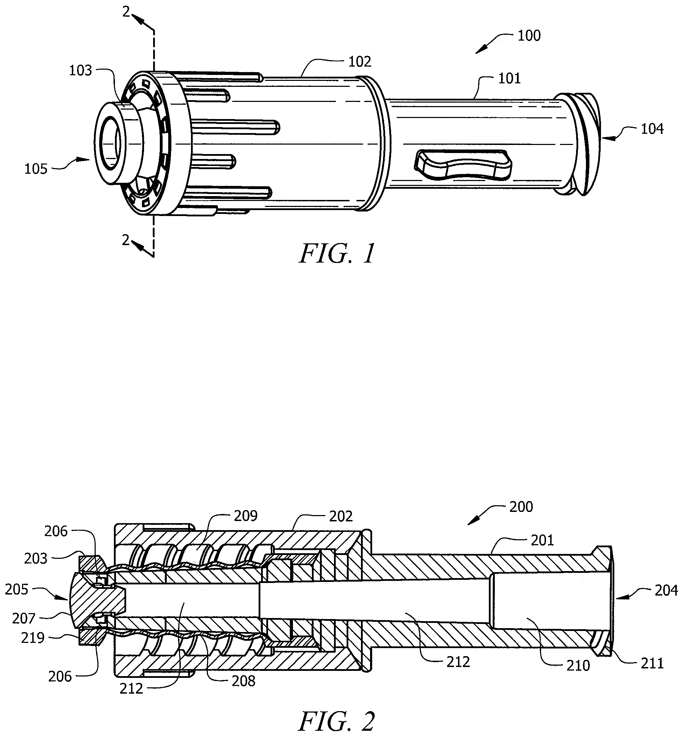

Of the drawings referred to above, only FIG. 2 shows the squeegee tip that constitutes an essential feature of the present invention.

### DETAILED DESCRIPTION OF THE INVENTION

Referring now to Figure 1, an embodiment of a medical connector 100 in accordance with the concepts described herein is shown. Medical connector 100 operates as a spin lock luer having a body 101 and a spin lock portion 102. The male luer body is ISO 594-2 compliant and interfaces with standard needless access devices. Medical connector 100 differs from traditional spin lock luer by the inclusion of a retractable elastic sleeve 103 over male luer portion 105 at the distal end of medical connector 100. The proximal end of medical connector 100 includes an inlet port 104 that can be connected to fluid delivery sources or devices such as IV fluid bags, pumps or the like.

As with traditional needleless medical connectors, male luer portion 105 of medical connector 100 is inserted into the female luer of another needleless access device to create a fluid path between a fluid delivery mechanism and a patient. In order to avoid drips and leakage of medical fluids that may be harmful to health care providers or patients, medical connector 100 is designed to minimize or eliminate fluid drips or leakage upon connection of medical connector 100 with another access device, or particularly upon disconnection from another access device with both devices are primed with fluid.

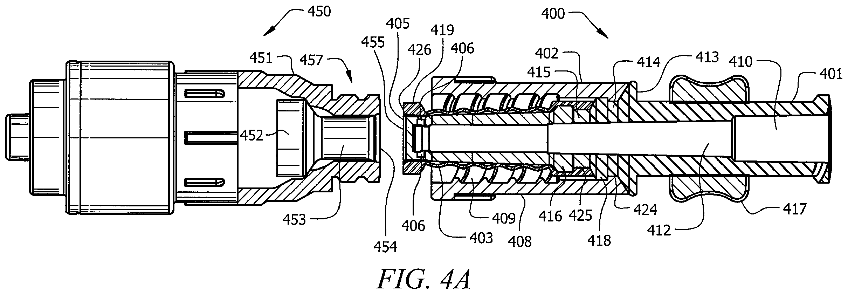

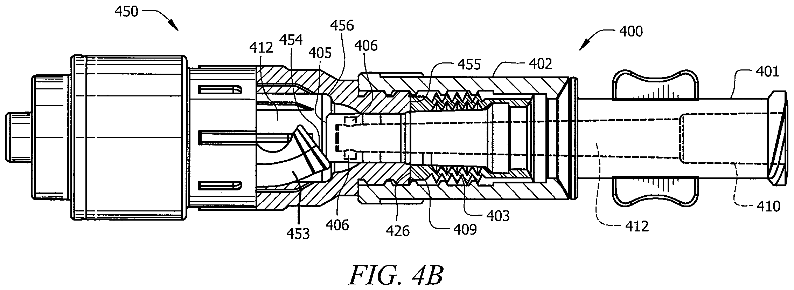

Referring now to Figure 2, the medical connector from Figure 1 is shown in a sectional view. As described, medical connector 200 includes body 201. Body 201 has an inlet port 204 adjacent to a threads 211 which allow connector 201 to be securely attached to other access devices. Inlet connection 210, which is shown here as a female luer connector that accepts the male luer of another needleless access devices and allows fluids to pass into fluid path 212. While connector 200 is shown as having a female luer type connector, any type of inlet port could be used, such as a bond pocket or other connector, while remaining within the scope of the concepts described herein. Fluid path 212 communicates with outlet ports 206 in male luer portion 205 of connector 200. Unlike other similar medical connectors in which the fluid path is directly out the end of the male luer, outlet ports 206 are channels in the sides of male luer portion 205 of body 201 and the tip of male luer portion 205 is closed. In embodiments of the medical connector according to the concepts described herein the tip of male luer portion 205 includes a squeegee tip 207, the function of which will be described with reference to Figure 4. Spin lock portion 202 surrounds the distal end of body 201 and includes inner chamber 209 which has threads for connecting medical connector 200 to other access devices.

Also surrounding male luer portion 205 of body 201 inside chamber 209 of spin lock portion 202 is flexible sleeve 203. Flexible sleeve 203 includes seal ring 219 at its distal end which operates to block outlet ports 206 when flexible sleeve 203 is in its extended position as shown in Figure 2. As will be discussed, flexible sleeve 203 can be pushed back into chamber 209 unblocking outlet ports 206 and completing the fluid path 212 through connector 200. In embodiments of flexible sleeve 203, sleeve portion 208 can be formed in an accordion shape to allow sleeve portion 208 to bunch together as seal ring 219 is pushed into chamber 209 by the engagement of connector 200 to another access device.

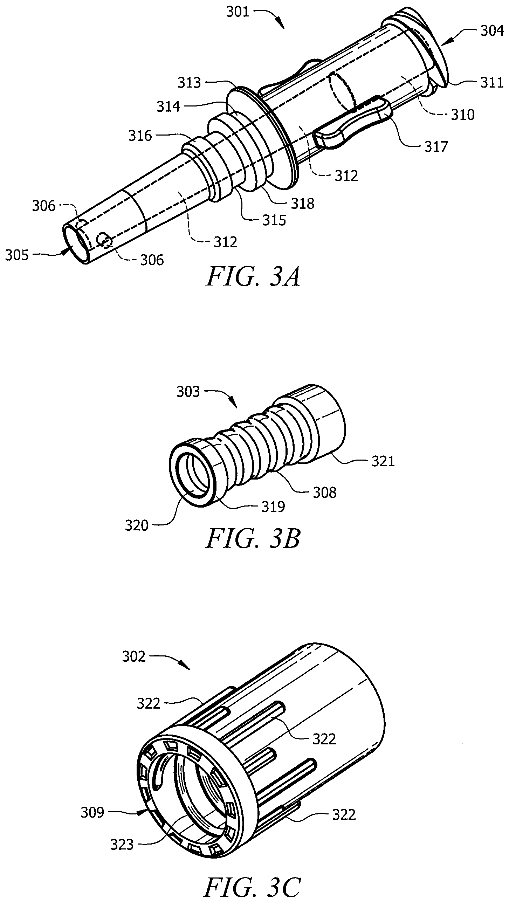

Referring now to Figures 3A, 3B and 3C embodiments of the body, flexible sleeve and spin lock portion, respectively, of a medical connector according to the concepts describe herein are shown. Figure 3A shows an embodiment of body 301. As described, body 301 includes inlet port 304 having connection mechanism 310, outlet ports 306, fluid path 312 and male luer portion 305. Threads 311 or other connection mechanisms can be placed at the proximal end of body 301 adjacent to inlet port 304. Grips 317 are used to facilitate manipulation of body 301 by health care personnel. Plate 313 works with flange 318 and groove 314 to hold spin lock portion 302 while allowing it to spin with respect to body 301. A flange in spin lock portion 302 fits snaps into groove 314 and spin lock portion is held in place by plate 313 and flange 318.

Similarly, flange 318, groove 315 and flange 316 are used to hold flexible sleeve 303 in position relative to body 301. Another flange inside end 321 of flexible sleeve 303 fits into groove 315 and is held in place by flanges 318 and 315, thereby holding flexible sleeve 303 in place. While flange and groove arrangements have been shown to attach spin lock portion 302 and flexible sleeve 303 to body 301, one skilled in the art would recognize that any number of arrangements could be employed to connect the various parts while still being within the scope of the concepts described herein. For example, adhesives, locking hubs, friction fittings or other connection mechanisms could be used to assemble the individual elements into a finished medical connector. Body 301 is preferably formed from polycarbonate plastic but could be formed from any number of materials appropriate for medical connectors.

An embodiment of flexible sleeve 303 is shown in Figure 3B. Flexible sleeve 303 includes end 321 which provides the attachment to body 301, sleeve portion 308 and seal ring 319. Inner surface 320 of seal ring 319 blocks outlet ports 306 of body 301 when flexible sleeve 303 is in its extended position. Seal ring 319 of flexible sleeve 303 may be pushed toward end 321 as will be described with reference to Figure 4B causing flexible sleeve 303 to shorten or compress along sleeve portion 308. Sleeve portion 308 may be formed in an accordion shape to allow sleeve portion 308 to bunch together or fold up as flexible sleeve 303 is compressed, or sleeve portion 308 may be merely formed of a compressible material, or otherwise formed or shaped to allow compression and shortening of sleeve portion 308.

As flexible sleeve 303 is shortened, inner surface 320 of seal ring 319 is moved away from outlet ports 306 causing them to be unblock and thereby opening a flow path through body 301. Since embodiments of male luer portion 305 of body 301 may be tapered, the inner diameter of flexible may need to be tapered as well to ensure that inner surface 320 is tight enough against outlet ports 306 to prevent fluid from escaping. Because of this taper, seal ring 319 and sleeve portion 308 should be able to expand as flexible sleeve 303 is compressed. This expansion or stretching of flexible sleeve 303 has the benefit of aiding in the return of flexible sleeve 303 to the extended position when the compression force to seal ring 309 is removed. Flexible sleeve 303 is preferably made from medical grade silicon, but can be made from any material that has the characteristics described with respect to flexible sleeve 303.

Spin lock portion 302 is designed to snap over body 301 as described above. Spin lock portion includes chamber 309 which holds male luer portion 305 of body 301 and flexible sleeve 303. Threads 323 on the inner surface of chamber 309 allow for secure connection to other threaded medical access devices. Grip rails 322 allow spin lock portion 302 to be firmly gripped by health care personnel during twisting motions required to thread or unthread spin lock portion 302 onto or off of another medical access device.

Referring now to Figures 4A and 4B, an embodiment of a medical connector 400 is shown in relation to another medical access device 450 to illustrate the operation of connector 400. Figure 4A shows connector 400 disengaged from medical access device 450 while Figure 4B shows connector 400 engaged with medical access device 450, creating a fluid path therethrough. Reference will be made to Figures 4A and 4B interchangeably as the operation of connector 400 is described.

Connector 400 includes body 401 having an inlet connection mechanism 410, spin lock portion 402 and flexible sleeve 403 as described above. Spin lock portion 402 is secured to body 401 by fitting spin lock flange 424 into groove 414 on body 401. Flange 418 and plate 413 hold spin lock portion 402 in place while allowing spin lock portion 402 to spin freely in relation to body 401. Flexible sleeve 403 has its distal end held in place on body 401 by flange 425 engaging with groove 415 and held in place by flange 416 on body 401. As described, seal ring 419 of flexible sleeve is positioned to block outlet ports 406 in male luer 405 of connector 400.

As can be seen in Figure 4A, when connector 400 is aligned with medical access device 401, surface 426 of seal ring 419 aligns to engage surface 455 of the female luer portion 457 of device 450. Further, mail luer portion 405 of connector 400 aligns with surface 454 of valve plug 453. Figure 4B show connector 400 engaged with device 450. In the engaged state, contact between surface 426 of flexible sleeve 403 and surface 455 of device 450 causes flexible sleeve to be compressed into chamber 409 formed by spin lock portion 402.

In the embodiment of flexible sleeve 403 shown, sleeve portion 408 folds along accordion bends, though other mechanisms to control the compression are well within the scope of the concepts described herein. As flexible sleeve 403 is compressed, it no longer blocks outlet valves 406. However, outlet valves 406 are then blocked by the inner surface of throat of female luer portion 457 of device 450.

As male luer portion 405 of connector 400 continues to be inserted into device 450, valve plug 453 compresses into device 450 and the tip of male luer portion 405 reaches chamber 456 where outlet ports 406 are no longer blocked and flow path 412 through connector 400 and medical access device 450 is completed. Connector 400 and device 450 may be securely attached using threads on spin lock portion 402 of connector 400 and threads on female luer portion 457 of device 450.

Upon disconnection, removing connector 400 from device 450 causes outlet ports 406 to again be blocked by the inner surface of the throat of female luer portion 457. This interrupts the fluid path 412 and stops the flow of fluid through connector 400. As connector 400 is disengaged, flexible sleeve 403 extends along male luer portion 405 and as outlet ports 406 are removed from device 450, they are immediately blocked by seal ring 419. A squeegee tip 207 from Figure 2 can be used to draw excess fluid from the throat of device 450 into the space between flexible sleeve 403 and male luer portion 405 as connector 400 is withdrawn.

While access device 450 is shown as having a valve plug arrangement, connector 400 will work with any standard female luer of a medical access device including bellows type plugs, devices with septums, or other configurations designed to accept standardized male luer connectors.

Although the present invention and its advantages have been described in detail, it should be understood that various changes, substitutions and alterations can be made herein without departing from the scope of the invention as defined by the appended claims.

## Claims

1. A medical connector (100) comprising:
a body having an inlet port (104), at least one outlet port (206) adjacent to a male luer portion (105) of the body, and a fluid path (212) between the inlet port (104) and the at least one outlet port (206);
and a retractable seal (219) adjacent to the male luer portion (105) of the body and blocking the at least one outlet port (206) of the body, when the male luer portion (105) of the medical connector is in a disconnected state; and
wherein the retractable seal (219) is positioned on the body such that it is configured to be movable away from the at least one outlet port (206) upon insertion of the male luer portion (105) of the medical connector (100) into a medical access device, whereby the at least one outlet port (206) is unblocked and a fluid path (212) through the medical connector (100) is opened,
**characterised in that**
the medical connector further comprises a squeegee tip (207) fixed to an end of the male luer portion (105), wherein the squeegee tip (207) is proximal to the at least one outlet port (206).

2. The medical connector (100) of claim 1 wherein the retractable seal (219) is part of a flexible sleeve (203) surrounding the male luer portion (105) of the body and fixed to an end of the male luer portion (105) of the body distal from the at least one outlet port (206).

3. The medical connector (100) of claim 2 wherein the flexible sleeve (203) compresses along the male luer portion (105) upon insertion into the medical access device.

4. The medical connector of claim 3, wherein:
the retractable seal is a seal ring (219) proximal to the at least one outlet port (206) of the body and operable to seal the at least one outlet port (206) when the flexible sleeve (203) is in an extended state, and wherein, when the male luer portion (105) of the body is inserted into the medical access device, the compression causes the seal ring (219) to be moved away from the outlet ports (206) along the male luer portion (105) of the body thereby unsealing the outlet ports (206) and creating fluid path (212) through the connector.

5. The medical connector (100) of claim 1 or 4, further comprising a spin lock (202) attached to the body, the spin lock (202) including threads for attachment to the medical access device.

6. The medical connector (100) of claim 1, wherein the squeegee tip removes excess fluid from the medical access device and causes the excess fluid to be trapped in the medical connector by the retractable seal.

7. The medical connector (100) of claim 3 or 4, further comprising a squeegee tip (207) fixed to an end of the male luer portion (105) proximal to the outlet ports (206), wherein the squeegee tip (207) removes excess fluid from the medical access device and causes the excess fluid to be trapped between the flexible sleeve (203) and the male luer portion (105) of the body.

8. The medical connector (100) of claim 1 or 4, wherein the at least one outlet port (206) are formed in side walls of the male luer portion (105) of the body.

9. A method of connecting a connector (100) and an access device to create a fluid path (212) for medical fluids, the method comprising:
inserting a male luer portion (105) of the connector (100) into a female luer portion (457) of the access device;
causing a seal blocking outlet ports (206) in the male luer portion (105) of the connector (100) to be retracted from the outlet ports (206);
creating a fluid path (212) through the connector (100) when the male luer portion (105) of the connector (100) has been inserted sufficiently into the access device to create a fluid path (212) from the connector (100) through the outlet ports (206) into the access device; and
causing the seal to reseal the outlet ports (206) upon removal of the male luer portion (105) of the connector (100) from the female luer portion (457) of the access device,
**characterised in that** the connector (100) includes a squeegee tip (207) fixed to an end of the male luer portion (105), wherein the squeegee tip (207) is proximal to the outlet ports (206).

10. The method of claim 9, wherein the retractable seal is part of a flexible sleeve (203) surrounding the male luer portion (105) of the body and fixed to an end of the male luer portion (105) of the body distal from the outlet ports (206).

11. The method of claim 10, wherein causing the seal to be retracted includes compressing the flexible sleeve (203) along the male luer portion (105) upon insertion into the medical access device.

12. The method of claim 9, further comprising removing excess fluid from the medical access device using the squeegee tip (207) and causing the excess fluid to be trapped in the medical connector (100) by the retractable seal.

13. The method of claim 12 wherein the retractable seal is part of a flexible sleeve (203) surrounding the male luer portion (105) of the connector (100) and fixed to an end of the male luer portion (105) distal from the outlet ports (206) and the excess fluid is trapped between the flexible sleeve (203) and the male luer portion (105).

## Patentansprüche

1. Medizinischer Verbinder (100), umfassend:
einen Körper mit einer Einlassöffnung (104), mindestens einer Auslassöffnung (206) angrenzend an einen Luer-Steckabschnitt (105) des Körpers, und einem Fluidpfad (212) zwischen der Einlassöffnung (104) und der mindestens einen Auslassöffnung (206);
und eine einziehbare Dichtung (219) angrenzend an den Luer-Steckabschnitt (105) des Körpers, die die mindestens eine Auslassöffnung (206) des Körpers blockiert, wenn sich der Luer-Steckabschnitt (105) des medizinischen Verbinders in einem nicht eingesteckten Zustand befindet; und
wobei die einziehbare Dichtung (219) am Körper so positioniert ist, dass sie dazu ausgelegt ist, beim Einsetzen des Luer-Steckabschnitts (105) des medizinischen Verbinders (100) in eine medizinische Zugangsvorrichtung von der mindestens einen Auslassöffnung (206) wegbewegbar zu sein, wodurch die mindestens eine Auslassöffnung (206) freigegeben und ein Fluidpfad (212) durch den medizinischen Verbinder (100) geöffnet wird,
**dadurch gekennzeichnet, dass**
der medizinische Verbinder ferner eine Abstreifspitze (207) aufweist, die an einem Ende des Luer-Steckabschnitts (105) befestigt ist, wobei die Abstreifspitze (207) proximal zur mindestens einen Auslassöffnung (206) angeordnet ist.

2. Medizinischer Verbinder (100) nach Anspruch 1, wobei die einziehbare Dichtung (219) Teil einer flexiblen Muffe (203) ist, die den Luer-Steckabschnitt (105) des Körpers umgibt und an einem Ende des Luer-Steckabschnitts (105) des Körpers distal zur mindestens einen Auslassöffnung (206) befestigt ist.

3. Medizinischer Verbinder (100) nach Anspruch 2, wobei die flexible Muffe (203) beim Einsetzen in die medizinische Zugangsvorrichtung entlang des Luer-Steckabschnitts (105) zusammengedrückt wird.

4. Medizinischer Verbinder nach Anspruch 3, wobei:
die einziehbare Dichtung ein Dichtungsring (219) proximal zur mindestens einen Auslassöffnung (206) des Körpers angeordnet und dahingehend funktionsfähig ist, die mindestens eine Auslassöffnung (206) abzudichten, wenn sich die flexible Muffe (203) in einem ausgedehnten Zustand befindet, und wobei, wenn der Luer-Steckabschnitt (105) des Körpers in die medizinische Zugangsvorrichtung eingeführt wird, das Zusammendrücken bewirkt, dass sich der Dichtring (219) von den Auslassöffnungen (206) entlang des Luer-Steckabschnitts (105) des Körpers wegbewegt, wodurch die Auslassöffnungen (206) freigegeben werden und ein durch den Verbinder verlaufender Fluidpfad (212) erzeugt wird.

5. Medizinischer Verbinder (100) nach Anspruch 1 oder 4, ferner umfassend eine am Körper befestigte Drehverriegelung (202), wobei die Drehverriegelung (202) ein Gewinde zur Anbringung an der medizinischen Zugangsvorrichtung beinhaltet.

6. Medizinischer Verbinder (100) nach Anspruch 1, wobei die Abstreifspitze überschüssige Flüssigkeit aus der medizinischen Zugangsvorrichtung entfernt und bewirkt, dass die überschüssige Flüssigkeit durch die einziehbare Dichtung in dem medizinischen Verbinder eingeschlossen wird.

7. Medizinischer Verbinder (100) nach Anspruch 3 oder 4, ferner umfassend eine Abstreifspitze (207), die an einem Ende des Luer-Steckabschnitts (105) proximal zu den Auslassöffnungen (206) befestigt ist, wobei die Abstreifspitze (207) überschüssige Flüssigkeit aus der medizinischen Zugangsvorrichtung entfernt und bewirkt, dass die überschüssige Flüssigkeit zwischen der flexiblen Muffe (203) und dem Luer-Steckabschnitt (105) des Körpers eingeschlossen wird.

8. Medizinischer Verbinder (400) nach Anspruch 1 oder 4, wobei die mindestens eine Auslassöffnung (206) in Seitenwänden des Luer-Steckabschnitts (105) des Körpers ausgebildet ist.

9. Verfahren zum Anschließen eines Verbinders (100) an eine Zugangsvorrichtung, um einen Fluidpfad (212) für medizinische Flüssigkeiten zu erzeugen, wobei das Verfahren umfasst:
Einsetzen eines Luer-Steckabschnitts (105) des Verbinders (100) in einen Luer-Aufnahmeabschnitt (457) der Zugangsvorrichtung;
Bewirken, dass eine Dichtung, die Auslassöffnungen (206) im Luer-Steckabschnitt (105) des Verbinders (100) blockiert, von den Auslassöffnungen (206) zurückgezogen wird;
Erzeugen eines durch den Verbinder (100) führenden Fluidpfades (212), wenn der Luer-Steckabschnitt (105) des Verbinders (100) ausreichend weit in die Zugangsvorrichtung eingeführt wurde, um einen Fluidpfad (212) vom Verbinder (100) durch die Auslassöffnungen (206) in die Zugangsvorrichtung zu erzeugen; und
Bewirken, dass die Dichtung die Auslassöffnungen (206) beim Entfernen des Luer-Steckabschnitts (105) des Verbinders (100) aus dem Luer-Aufnahmeabschnitt (457) der Zugangsvorrichtung wieder verschließt,
**dadurch gekennzeichnet, dass** der Verbinder (100) eine Abstreifspitze (207) beinhaltet, die an einem Ende des Luer-Steckabschnitts (105) befestigt ist, wobei die Abstreifspitze (207) proximal zu den Auslassöffnungen (206) angeordnet ist.

10. Verfahren nach Anspruch 9, wobei die einziehbare Dichtung Teil einer flexiblen Muffe (203) ist, die den Luer-Steckabschnitt (105) des Körpers umgibt und an einem Ende des Luer-Steckabschnitts (105) des Körpers distal zu den Auslassöffnungen (206) befestigt ist.

11. Verfahren nach Anspruch 10, wobei das Bewirken, dass die Dichtung zurückgezogen wird, das Zusammendrücken der flexiblen Muffe (203) entlang des Luer-Steckabschnitts (105) beim Einsetzen in die medizinische Zugangsvorrichtung umfasst.

12. Verfahren nach Anspruch 9, ferner umfassend das Entfernen von überschüssiger Flüssigkeit aus der medizinischen Zugangsvorrichtung unter Verwendung der Abstreifspitze (207) und das Bewirken, dass die überschüssige Flüssigkeit im medizinischen Verbinder (100) durch die einziehbare Dichtung eingeschlossen wird.

13. Verfahren nach Anspruch 12, wobei die einziehbare Dichtung Teil einer flexiblen Muffe (203) ist, die den Luer-Steckabschnitt (105) des Verbinders (100) umgibt und an einem Ende des Luer-Steckabschnitts (105) distal zu den Auslassöffnungen (206) befestigt ist und die überschüssige Flüssigkeit zwischen der flexiblen Muffe (203) und dem Luer-Steckabschnitt (105) eingeschlossen wird.

## Revendications

1. Connecteur médical (100) comprenant :
un corps présentant un orifice d'entrée (104), au moins un orifice de sortie (206) adjacent à une partie Luer mâle (105) du corps, et un trajet de fluide (212) entre l'orifice d'entrée (104) et l'au moins un orifice de sortie (206) ;
et un joint d'étanchéité rétractable (219) adjacent à la partie Luer mâle (105) du corps et bloquant l'au moins un orifice de sortie (206) du corps lorsque la partie Luer mâle (105) du connecteur médical est dans un état déconnecté ; et
sachant que le joint d'étanchéité rétractable (219) est positionné sur le corps de telle façon qu'il soit configuré pour pouvoir être écarté de l'au moins un orifice de sortie (206) lors de l'insertion de la partie Luer mâle (105) du connecteur médical (100) dans un dispositif d'accès médical, suite à quoi l'au moins un orifice de sortie (206) est débloqué et un trajet de fluide (212) à travers le connecteur médical (100) est ouvert,
**caractérisé en ce que**
le connecteur médical comprend en outre une pointe de racloir (207) fixée à une extrémité de la partie Luer mâle (105), sachant que la pointe de racloir (207) est située de manière proximale par rapport à l'au moins un orifice de sortie (206).

2. Le connecteur médical (100) de la revendication 1, sachant que le joint d'étanchéité rétractable (219) fait partie d'un manchon flexible (203) entourant la partie Luer mâle (105) du corps et est fixé à une extrémité de la partie Luer mâle (105) du corps de manière distale par rapport à l'au moins un orifice de sortie (206).

3. Le connecteur médical (100) de la revendication 2, sachant que le manchon flexible (203) se comprime le long de la partie Luer mâle (105) lors de l'insertion dans le dispositif d'accès médical.

4. Le connecteur médical (100) de la revendication 3, sachant que :
le joint d'étanchéité rétractable est une bague d'étanchéité située de manière proximale par rapport à l'au moins un orifice de sortie (206) du corps et utilisable pour obturer l'au moins un orifice de sortie (206) lorsque le manchon flexible (203) est dans un état étendu, et sachant que, lorsque la partie Luer mâle (105) du corps est insérée dans le dispositif d'accès médical, la compression fait en sorte que la bague d'étanchéité (219) s'écarte des orifices de sortie (206) le long de la partie Luer mâle (105) du corps en désobturant les orifices de sortie (206) et créant un trajet de fluide (212) à travers le connecteur.

5. Le connecteur médical (100) de la revendication 1 ou 4, comprenant en outre un verrou de type spinlock (202) attaché au corps, le verrou de type spinlock (202) incluant des filetages pour la fixation au dispositif d'accès médical.

6. Le connecteur médical (100) de la revendication 1, sachant que la pointe de racloir enlève du fluide excédentaire du dispositif d'accès médical et fait en sorte que le fluide excédentaire soit piégé dans le connecteur médical par le joint d'étanchéité rétractable.

7. Le connecteur médical (100) de la revendication 3 ou 4, comprenant en outre une pointe de racloir (207) fixée à une extrémité de la partie Luer mâle (105) de manière proximale par rapport aux orifices de sortie (206), sachant que la pointe de racloir (207) enlève du fluide excédentaire du dispositif d'accès médical et fait en sorte que le fluide excédentaire soit piégé entre le manchon flexible (203) et la partie Luer mâle (105) du corps.

8. Le connecteur médical (100) de la revendication 1 ou 4, sachant que l'au moins un orifice de sortie (206) est formé dans des parois latérales de la partie Luer mâle (105) du corps.

9. Procédé de connexion d'un connecteur (100) et d'un dispositif d'accès pour créer un trajet de fluide (212) pour fluides médicaux, le procédé comprenant :
l'insertion d'une partie Luer mâle (105) du connecteur (100) dans une partie Luer femelle (457) du dispositif d'accès ;
le fait de faire en sorte qu'un joint d'étanchéité bloquant des orifices de sortie (206) dans la partie Luer mâle (105) du connecteur (100) se rétracte des orifices de sortie (206) ;
le fait de créer un trajet de fluide (212) à travers le connecteur (100) lorsque la partie Luer mâle (105) du connecteur (100) a été suffisamment insérée dans le dispositif d'accès pour créer un trajet de fluide (212) depuis le connecteur (100) à travers les orifices de sortie (206) jusqu'au dispositif d'accès ; et
le fait de faire en sorte que le joint d'étanchéité réobture les orifices de sortie (206) lors de l'enlèvement de la partie Luer mâle (205) du connecteur (100) de la partie Luer femelle (457) du dispositif d'accès,
**caractérisé en ce que** le connecteur (100) inclut une pointe de racloir (207) fixée à une extrémité de la partie Luer mâle (105), sachant que la pointe de racloir (207) est située de manière proximale par rapport aux orifices de sortie (206).

10. Le procédé de la revendication 9, sachant que le joint d'étanchéité rétractable fait partie d'un manchon flexible (203) entourant la partie Luer mâle (105) du corps et est fixé à une extrémité de la partie Luer mâle (105) du corps de manière distale par rapport aux orifices de sortie (206).

11. Le procédé de la revendication 10, sachant que le fait de faire se rétracter le joint d'étanchéité inclut la compression du manchon flexible (203) le long de la partie Luer mâle (105) lors de l'insertion dans le dispositif d'accès médical.

12. Le procédé de la revendication 9, comprenant en outre l'enlèvement de fluide excédentaire du dispositif d'accès médical au moyen de la pointe de racloir (207) et le fait de faire en sorte que le fluide excédentaire soit piégé dans le connecteur médical (100) par le joint d'étanchéité rétractable.

13. Le procédé de la revendication 12, sachant que le joint d'étanchéité rétractable fait partie d'un manchon flexible (203) entourant la partie Luer mâle (105) du connecteur (100) et est fixé à une extrémité de la partie Luer mâle (105) de manière distale par rapport aux orifices de sortie (206) et le fluide excédentaire est piégé entre le manchon flexible (203) et la partie Luer mâle (105).
